# EUROPEAN PATENT SPECIFICATION

(11) **EP 4 368 159 B1**
(45) Date of publication and mention of the grant of the patent: **23.09.2026**
(21) Application number: 22837979.8
(22) Date of filing: 06.07.2022
(51) Int. Cl.: A61F 13/06, A61F 13/02, A61F 13/00, A61F 5/11, A61F 13/0246

(54) **TOENAIL CARE TAPE**
ZEHENNAGELPFLEGEBAND
BANDE DE SOIN D'ONGLE D'ORTEIL

(30) Priority: 07.07.2021 KR 20210089182
(43) Date of publication of application: 15.05.2024
(73) Proprietor: Hong, Tae Kyung, Incheon 22760 (KR)
(72) Inventor: Hong, Tae Kyung, Incheon 22760 (KR)
(74) Representative: Caldon, Giuliano
(86) International application number: PCT/KR2022/009774
(87) International publication number: WO 2023/282627

(56) References cited:
- CN-U- 208 243 673
- JP-A- 2000 300 601
- JP-A- 2005 013 297
- JP-A- 2005 073 977
- JP-A- 2007 159 825
- JP-A- 2017 064 204
- JP-A- H 045 959
- KR-A- 20130 139 513
- KR-B1- 102 335 643
- US-A- 3 464 408

## Description

### [TECHNICAL FIELD]

The present invention relates to a toenail care tape, in more detail, the toenail care tape pulls down the surrounding flesh of a splinted ingrown toenail so that the toenail can be easily and properly lifted and cares the toenail to grow normally by taping the surrounding flesh of an athlete's foot toenail with scaling care.

### [BACKGROUND ART]

As is well known, an ingrown toenail (hereinafter referred to as 'ingrown toenail') is a disease in which the fingernail or toenail digs into the flesh, causing inflammation and pain, and it mainly occurs in the big toenail.

The ingrown toenail can be caused by any situation where the flesh on the outside of the toenail is continuously pressed. In particular, the ingrown toenail occurs when the outside of the toenail is deeply cut by nail clippers and uncut toenail fragments hidden in the flesh dig into the flesh, when toenail fungus is left for a long time and the shape of the toenail is deformed and tight shoes are worn for a long time, when the toenail bone protrudes and internal pressure increases, or when the toenail naturally becomes more curved due to obesity or aging. And if family members have ingrown toenails, other family members tend to be more prone to it, so genetic factors are also thought to be involved.

The ingrown toenail most commonly occurs on the big toe of the five toes. In particular, the ingrown toenail commonly occurs on the big toe of the right foot. This is probably because it is the area that experiences the most pressure when walking or running. At first, the outside or inside of the big toe becomes slightly red, swollen, and causes mild pain. Soon, as the friction increases, it becomes more swollen and oozing, granulation tissue (a lump of inflammation, blood vessels, and fibrous tissue) proliferates, and the area around the toenail begins to fester. As the symptoms progress, the smell becomes strong, the pain becomes severe, and normal walking becomes difficult.

If walking difficulties and inflammation caused by this pain become severe, secondary infectious diseases such as cellulitis (a disease in which bacteria invade subcutaneous tissue and cause purulent inflammation) may occur.

As described above, as a prior art for treating the ingrown toenail, Republic of Korea Patent No. 10-2181782 (registration date November 17, 2020) 'Prosthesis for ingrown toenail', Republic of Korea published patent no. 10 -2018-0079853 (publication date: July 11, 2018) 'Ingrown toenail correction device', Republic of Korea registered patent No. 10-2253306 (registration date: May 12, 2021) 'Ingrown toenail correction device' and Korea public patent no. No. 10-2021-0023330 (publication date March 4, 2021) 'Ingrown toenail correction device' etc. are disclosed. Document CN 208243673 U, which discloses the preamble of claim 1, describes a known toenail care tape comprising a connecting strip, two V-shaped traction tabs extending form the connecting strip, and two reinforcement portions extending from the lateral side of the connecting strip. Document KR 20130139513 A describes a known tape to facilitate the application of manicures. This tape comprises a flat body in the form of an adhesive sheet, the lower surface of which is coated with an adhesive. The tape has a U-shape, with two arms that define a concave recess that is shaped to match the shape of the nail. In use, the user removes a protective film covering the adhesive, applies the tape around the nail, and proceeds with applying nail polish. Document JP H045959 A describes a protective tape for fingertips, for example, during sports activities. This known tape comprises an upper portion with a central slit. This slit allows the upper portion to be split into two tabs, which, when applied to the finger, can cross over the nail, adapting to the curvature of the finger. The adhesive tape also comprises a horizontal lower portion, which is designed to be wrapped around the finger. Document US 3464408 A describes a tape for relieving discomfort caused by an ingrown toenail. Specifically, this known tape comprises an adhesive base with two protruding portions, from which two converging strips extend, terminating in a common end, thus defining a triangular shape. During application, the tape is slid toward the back of the finger, so that the converging strips progressively insert themselves under the corners of the nail, until reaching the desired depth. The common end of the converging strips is then pressed downward, while the adhesive base and the protruding portions are fixed to the upper surface of the finger.

However, the above-described prior art treats toenails that have already dug into the flesh, and has the problem of not being able to prevent the toenails from digging into the flesh when they grow out.

In addition, the above-described prior art has a complicated structure and difficult operation method, so it requires the help of an expert, so there is a problem in that the user cannot easily use it directly.

On the other hand, if the flesh in front of the toes fills up after scaling treatment, athlete's foot toenails interfere with the normal growth of the toenails, causing the toenails to grow convex or extend upward.

### [DISCLOSURE]

### [TECHNICAL PROBLEM]

The present invention was devised to solve the conventional problems described above, an object of the present disclosure is to provide the toenail care tape pulling down the surrounding flesh of a splinted ingrown toenail so that the toenail can be easily and properly lifted and caring the toenail to grow normally by taping the surrounding flesh of an athlete's foot toenail with scaling care.

### [TECHNICAL SOLUTION]

In order to achieve the above object, the technical idea of the present invention is to Comprise an edge adhesive part adhered along the lower flesh and inner and outer flesh, which are the peripheral edges of the toenail, a center adhesive part connected to the bottom center of the edge adhesive part and adhered to the underside of the toes to tension the lower flesh and a side adhesive part by connected to both sides of the bottom of the edge adhesive part and passed through the bottom of the toes and adhered to the upper surface of toes adjacent to the toenail to tension the inner and outer flesh.

The edge adhesive part comprises a lower flesh adhesive part adhered to the lower flesh of the toenail, an inner flesh adhesive part adhered to the lower flesh of the toenail and an outer flesh adhesive part adhered to the outer flesh of the toenail.

The side adhesive part comprises an inner adhesive part connected to the lower end of the inner flesh adhesive part and an outer adhesive part connected to the lower end of the outer flesh adhesive part.

At this time, the ratio of the horizontal widths of the inner adhesive part, center adhesive part and outer adhesive part is 1:3:1.

The ratio of the vertical lengths of the edge adhesive part 110, the center adhesive part, and the side adhesive part is 1:2:4.

### [ADVANTAGEOUS EFFECT]

According to the present invention which is implemented as a solution described above, the lower flesh and inner and outer flesh of the toe at the peripheral edge of the toenail are pulled downward and tense, thereby preventing the toenail from digging into the flesh when growing, thereby improving blood circulation in the toe, since the process of adhering the tape to the edge around the toenail is simple, making it very easy for users to use.

### [DESCRIPTION OF DRAWINGS]

Fig. 1 is a perspective view showing the overall shape according to an embodiment of the present invention.
Fig. 2 is a front view showing the front according to an embodiment of the present invention.
Figure 3 is a plan view showing the state of use in which the tape according to an embodiment of the present invention is adhered to the big toe.
Fig. 4 to 8 are illustrations showing the tape adhesion process from the front according to an embodiment of the present invention.

### [BEST MODE OF THE DISCLOSURE]

In order to fully understand the present invention, preferred embodiments of the present invention will be described with reference to the accompanying drawings.

Embodiments of the present invention may be modified in various forms, and the scope of the present invention should not be construed as limited to the embodiments described in detail below. This embodiment is provided to more completely explain the present invention to those skilled in the art.

Therefore, the shapes of components expressed in the drawings may be exaggerated to emphasize a clearer description. It should be noted that identical members in each drawing may be indicated by the same reference numerals. Additionally, detailed descriptions of well-known functions and configurations that are judged to unnecessarily obscure the gist of the present invention are omitted.

Hereinafter, preferred embodiments of the present invention will be described in detail with reference to the attached drawings.

The toenail care tape 100 according to an embodiment of the present invention includes an edge adhesive part 110 adhered along the lower flesh 12 and inner and outer flesh 13,14 which are the peripheral edges of the toenail 11, a center adhesive part 120 connected to the bottom center of the edge adhesive part 110 and adhered to the underside of the toes 10 to tension the lower flesh 12, a side adhesive part connected to both sides of the bottom of the edge adhesive part 110 and passes through the bottom 16 of the toe 10 and adheres to the upper surface 15 of the toe 10 adjacent to the toenail 11 to tension the inner and outer flesh 13,14.

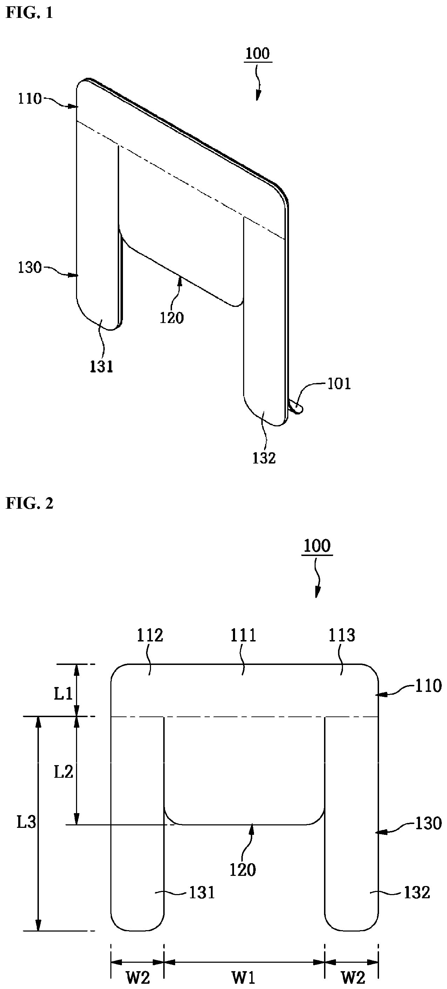

First, as shown in Figures 1 and 2, the toenail care tape 100 according to an embodiment of the present invention is made by cutting a square film coated with an adhesive to the back side and forming an edge adhesive part 110, center adhesive part 120, and side adhesive part 130 as a single body.

Alternatively, the toenail care tape 100 according to an embodiment of the present invention may be made up of a plurality of pieces so as to be continuously connected to a long sheet coated with adhesive, and may be stored in a rolled form on a roll.

In addition, the toenail care tape 100 according to an embodiment of the present invention is made of cotton, polyurethane, and non-woven materials to have a predetermined elasticity.

In addition, in the toenail care tape 100 according to an embodiment of the present invention, a release paper 101 is attached to the adhesive surface coated with the adhesive to protect the adhesive surface.

The edge adhesive part 110 is adhered along the lower flesh 12 of the toes 10 below the peripheral edge of the toenail 11 and the inner and outer flesh 13, 14 of the toes 10 on the inner and outer sides.

Referring to Figures 1 and 2, the edge adhesive part 110 is formed in a rectangular shape elongated in the left-right direction.

The edge adhesive part 110 has a lower flesh adhesive part 111 adhered to the lower flesh 12 of the toenail 11 in the center, an inner flesh adhesive part 112 adhered to the inner flesh 13 of the toenail 11 on the left side corresponding to the inner side (IS) of the toes 10, and an outer flesh adhesive part 113 adhered to the outer flesh 14 of the toenail 11 on the right side corresponding to the outer side (OS) of the toes 10.

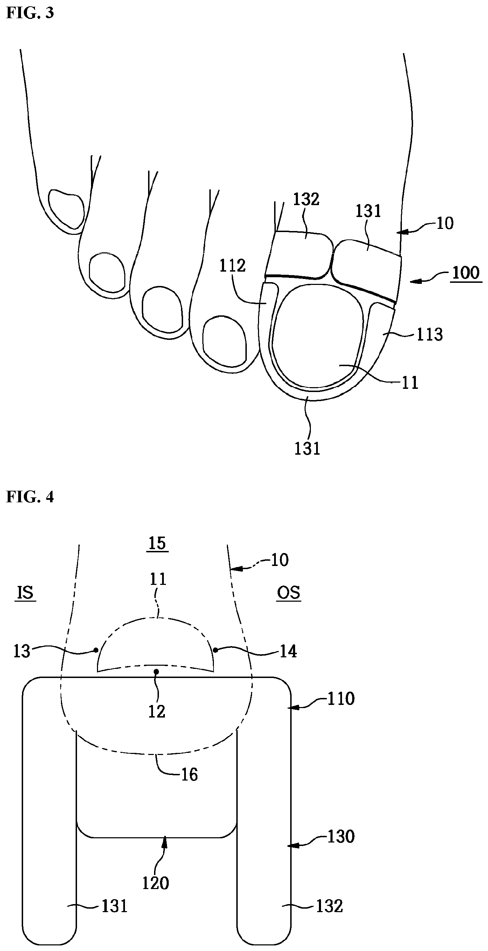

As shown in FIG. 3, this edge adhesive part 110 is adhered along the lower flesh 12 and the inner and outer flesh 13,14 so as to be close to the toenail 11.

The center adhesive part 120 is adhered to the underside of the toes 10, and the lower flesh adhesive portion 111 functions to pull down and tension the lower flesh 12 of the toes 10.

Referring to Figures 1 and 2, the center adhesive part 120 is formed in a rectangular shape and is connected to the lower end of the lower flesh adhesive part (111).

The vertical length L2 of the center adhesive part 120 may be shorter than the vertical length L3 of the side adhesive part 130.

At this time, the ratio of the vertical length L1 of the edge adhesive part 110, the vertical length L2 of the center adhesive part 120, and the vertical length L3 of the side adhesive part 130 is preferably 1:2:4.

Alternatively, the vertical length L2 of the center adhesive part 120 may be formed to be the same or similar to the vertical length L3 of the side adhesive part 130.

As shown in FIG. 3, this center adhesive part 120 is adhered to the underside of the toes 10 and tensions the lower flesh adhesive part 111 by pulling it down.

The side adhesive part (130) is adhered to the upper surface (15) of the toes (10) and tensions the inner and outer flesh (13) (14) of the toes (10) by pulling it down.

Referring to Figures 1 and 2, the side adhesive part 130 is formed in a vertically elongated rectangular shape and includes an inner adhesive part 131 connected to the lower left side of the edge adhesive part 110 and an outer adhesive part 132 connected to the lower right side of the edge adhesive part 110.

Here, the vertical lengths of the inner adhesive part 131 and the outer adhesive part 132 are formed to be the same.

At this time, the ratio of the horizontal width of the inner adhesive part 131, the horizontal width of the center adhesive part 120, and the horizontal width of the outer adhesive part 132 is preferably 1:3:1.

Alternatively, the inner adhesive part 131 and the outer adhesive part 132 can be cut from both sides of the center adhesive part 120 using the perforated lines formed on both ends of the center adhesive part (120).

As shown in FIG. 3, the inner adhesive part 131 and the outer adhesive part 132 of the side adhesive part 130 passes through the bottom (16) of the toes (10) and adhere to the upper surface (15) of the toes (10) adjacent to the toenail (11) and tensions the inner flesh (13) and outer flesh (14) by pulling it down.

### [MODE OF THE DISCLOSURE]

The adhesion process of the tape 100 according to the embodiment of the present invention configured as described above will be described in detail with reference to the attached drawings as follows.

First, after peeling off the release paper 101 from the adhesive side of the tape 100, as shown in FIG. 4, the lower flesh adhesive part 111 is adhered to the lower flesh 12 of the toes 10 so as to be close to the toenail 11.

Next, the inner flesh adhesive part 112 and the outer flesh adhesive part 113 are adhered to the inner flesh 13 and the outer flesh 14 of the toes 10 so as to be close to the toenail 11.

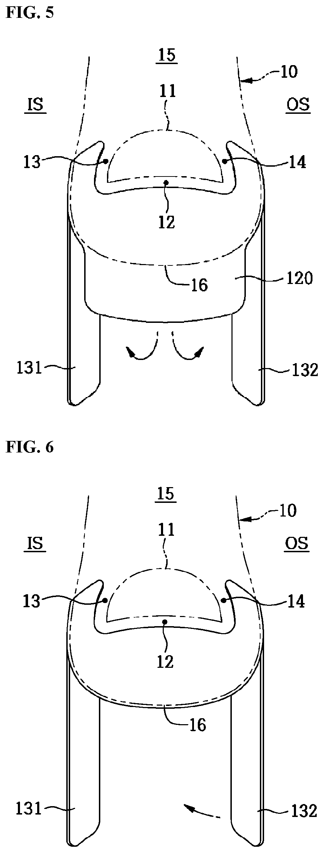

Next, as shown in FIG. 5, when the center adhesive part 120 is pulled in the direction of the arrow and adhered to the bottom 16 of the toes 10, the lower flesh adhesive part 111 pulls and tensions the lower flesh 12 of the toes 10.

Continuing, as shown in FIG. 6, when the outer adhesive part 132 is pulled in the direction of the arrow and passes through the bottom 16 of the toes 10 and is adhered to the upper surface 15 of the toes 10 adjacent to the toenail 11, the outer flesh adhesive part 113 pulls and tensions the outer flesh 14 of the toes 10.

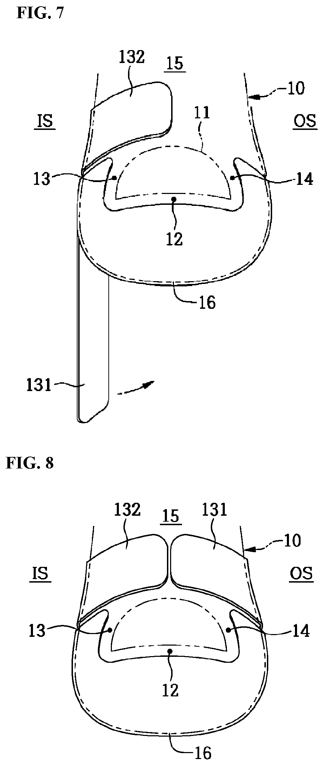

Finally, as shown in FIG. 7, the inner adhesive part 131 is pulled in the direction of the arrow and passed through the bottom 16 of the toes 10 and adhered to the upper surface 15 of the toes 10 adjacent to the toenail 11, the inner flesh adhesive part 112 pulls and tensions the inner flesh 13 of the toes 10.

At this time, the outer adhesive part 132 and the inner adhesive part 131 intersect in an 'X' shape on the bottom 16 of the toes 10.

Figure 8 shows the completed state in which the toenail care tape 100 according to an embodiment of the present invention is adhered to the big toe 10.

Therefore, according to an embodiment of the present invention, since the lower flesh 12 and inner and outer flesh 13 14 of the toes 10 at the peripheral edge of the toenail 11 is pulled down and tensioned, there is an advantage of preventing the toenail 11 from digging into the flesh as the toenail 11 grows.

In addition, according to the embodiment of the present invention, the process of peeling off the release paper 101 and then adhering the tape 100 to the edge around the toenail 11 is simple, so there is an advantage that the user can easily use it directly.

The embodiments of the present invention described above are merely illustrative, and those skilled in the art will understand that various modifications and other equivalent embodiments are possible.

Therefore, it will be understood that the present invention is not limited to the forms mentioned in the detailed description above.

## Claims

1. A toenail care tape (100) comprising;
an edge adhesive part (110) configured to be adhered along the lower flesh (12) and inner and outer flesh (13, 14), which are the peripheral edges of the toenail (11); wherein the edge adhesive part (110) is formed in a rectangular shape elongated in a left-right direction; wherein the edge adhesive part (110) has a lower flesh adhesive part (111) configured to be adhered to the lower flesh (12) of the toenail (11) in the center, an inner flesh adhesive part (112) configured to be adhered to the inner flesh (13) of the toenail (11) on the left side corresponding to the inner side (IS) of the toes (10), and an outer flesh adhesive part (113) configured to be adhered to the outer flesh (14) of the toenail (11) on the right side corresponding to the outer side (OS) of the toes (10);
a side adhesive part (130) connected to both sides of the lower end of the edge adhesive part (110) and configured to be passed through the bottom (16) of the toes (10) and adhered to the upper surface (15) of toes (10) adjacent to the toenail (11) to tension the inner and outer flesh (13, 14);
the toenail care tape (100) being **characterized in that** it further comprises a center adhesive part (120) connected to the center of the lower end of the edge adhesive part (110) and configured to be adhered to the underside of the toes (10) to tension the lower flesh (12);
wherein the center adhesive part (120) is formed in a rectangular shape and is connected to the lower end of the lower flesh adhesive part (111);
wherein the side adhesive part (130) is formed in a vertically elongated rectangular shape and includes:
- an inner adhesive part (131) connected to the lower left side of the edge adhesive part (110), and
- an outer adhesive part (132) connected to the lower right side of the edge adhesive part (110).

2. The toenail care tape (100) of claim 1, wherein the ratio of the horizontal widths of the inner adhesive part (131), center adhesive part (120) and outer adhesive part (132) is 1:3:1.

3. The toenail care tape (100) of claim 1, wherein the ratio of the vertical lengths of the edge adhesive part (110), the center adhesive part (120), and the side adhesive part (130) is 1:2:4.

## Patentansprüche

1. Zehennagelpflegeband (100), umfassend;
einen Randklebeteil (110), der darauf ausgelegt ist, entlang des unteren Fleisches (12) und des inneren und äußeren Fleisches (13, 14), die die Außenränder des Zehennagels (11) sind, verklebt zu werden; wobei der Randklebeteil (110) in einer rechteckigen Form ausgebildet ist, die sich in einer Links-Rechts-Richtung ausdehnt; wobei der Randklebeteil (110) einen unteren Fleisch-Klebeteil (111), der darauf ausgelegt ist, am unterem Fleisch (12) des Zehennagels (11) in der Mitte verklebt zu werden, einen inneren Fleisch-Klebeteil (112), der darauf ausgelegt ist, am inneren Fleisch (13) des Zehennagels (11) auf der linken Seite, die der inneren Seite (IS) der Zehen (10) entspricht, verklebt zu werden, und einen äußeren Fleisch-Klebeteil (113), der darauf ausgelegt ist, am äußeren Fleisch (14) des Zehennagels (11) auf der rechten Seite, die der Außenseite (OS) der Zehen (10) entspricht, verklebt zu werden, aufweist;
einen Seitenklebeteil (130), der mit beiden Seiten des unteren Endes des Randklebeteils (110) verbunden ist und darauf ausgelegt ist, über die Unterseite (16) der Zehen (10) durchgeführt und an der Oberseite (15) der Zehen (10), an den Zehennagel (11) angrenzend, verklebt zu werden, um das innere und äußere Fleisch (13, 14) zu spannen;
wobei das Zehennagelpflegeband (100) **dadurch gekennzeichnet ist, dass** es außerdem einen mittleren Klebeteil (120) umfasst, der mit der Mitte des unteren Endes des Randklebeteils (110) verbunden und darauf ausgelegt ist, an der Unterseite der Zehen (10) verklebt zu werden, um das untere Fleisch (12) zu spannen;
wobei der mittlere Klebeteil (120) in einer rechteckigen Form ausgebildet ist und mit dem unteren Ende des unteren Fleisch-Klebeteils (111) verbunden ist;
wobei der Seitenklebeteil (130) in einer vertikal ausgedehnten rechteckigen Form ausgebildet ist und Folgendes einschließt:
- einen inneren Klebeteil (131), der mit der unteren linken Seite des Randklebeteils (110) verbunden ist, und
- einen äußeren Klebeteil (132), der mit der unteren rechten Seite des Randklebeteils (110) verbunden ist.

2. Zehennagelpflegeband (100) nach Anspruch 1, wobei das Verhältnis der horizontalen Breiten des inneren Klebeteils (131), des mittleren Klebeteils (120) und des äußeren Klebeteils (132) 1:3:1 beträgt.

3. Zehennagelpflegeband (100) nach Anspruch 1, wobei das Verhältnis der vertikalen Längen des Randklebeteils (110), des mittleren Klebeteils (120) und des Seitenklebeteils (130) 1:2:4 beträgt.

## Revendications

1. Bande de soin d'ongle d'orteil (100) comprenant :
une partie adhésive de bord (110) configurée pour adhérer le long de la chair inférieure (12) et de la chair intérieure et extérieure (13, 14), qui sont les bords périphériques de l'ongle d'orteil (11) ; dans laquelle la partie adhésive de bord (110) est de forme rectangulaire allongée dans une direction gauche-droite ; dans laquelle la partie adhésive de bord (110) présente une partie adhésive de chair inférieure (111) configurée pour adhérer à la chair inférieure (12) de l'ongle d'orteil (11) au centre, une partie adhésive de chair intérieure (112) configurée pour adhérer à la chair intérieure (13) de l'ongle d'orteil (11) sur le côté gauche correspondant au côté intérieur (IS) des orteils (10), et une partie adhésive de chair extérieure (113) configurée pour adhérer à la chair extérieure (14) de l'ongle d'orteil (11) sur le côté droit correspondant au côté extérieur (OS) des orteils (10) ;
une partie adhésive latérale (130) reliée aux deux côtés de l'extrémité inférieure de la partie adhésive de bord (110) et configurée pour passer à travers le fond (16) des orteils (10) et adhérer à la surface supérieure (15) des orteils (10) adjacents à l'ongle d'orteil (11) pour tendre la chair intérieure et extérieure (13, 14) ;
la bande de soin d'ongle d'orteil (100) étant **caractérisée en ce qu'**elle comprend en outre une partie adhésive centrale (120) reliée au centre de l'extrémité inférieure de la partie adhésive de bord (110) et configurée pour adhérer à la face inférieure des orteils (10) afin de tendre la chair inférieure (12) ;
dans laquelle la partie adhésive centrale (120) est de forme rectangulaire et est reliée à l'extrémité inférieure de la partie adhésive de chair inférieure (111) ;
dans laquelle la partie adhésive latérale (130) présente une forme rectangulaire allongée verticalement et comprend :
- une partie adhésive intérieure (131) reliée au côté inférieur gauche de la partie adhésive de bord (110), et
- une partie adhésive extérieure (132) reliée au côté inférieur droit de la partie adhésive de bord (110).

2. Bande de soin d'ongle d'orteil (100) selon la revendication 1, dans laquelle le rapport entre les largeurs horizontales de la partie adhésive intérieure (131), de la partie adhésive centrale (120) et de la partie adhésive extérieure (132) est de 1:3:1.

3. Bande de soin d'ongle d'orteil (100) selon la revendication 1, dans laquelle le rapport entre les longueurs verticales de la partie adhésive de bord (110), de la partie adhésive centrale (120) et de la partie adhésive latérale (130) est de 1:2:4.
